(19) **Europäisches Patentamt**

**European Patent Office**

**Office européen des brevets**

(11) **EP 1 303 771 B1**

(12) **EUROPÄISCHE PATENTSCHRIFT**

(45) Veröffentlichungstag und Bekanntmachung des Hinweises auf die Patenterteilung:
**07.12.2005 Patentblatt 2005/49**

(51) Int Cl.⁷: **G01V 3/08**, A61B 5/06, G01B 7/004

(21) Anmeldenummer: **01944861.2**

(22) Anmeldetag: **10.07.2001**

(86) Internationale Anmeldenummer:
**PCT/CH2001/000431**

(87) Internationale Veröffentlichungsnummer:
**WO 2002/008793 (31.01.2002 Gazette 2002/05)**

(54) **VERFAHREN ZUR BESTIMMUNG DER POSITION EINES SENSORELEMENTES**

METHOD FOR DETERMINING THE POSITION OF A SENSOR ELEMENT

PROCEDE POUR DETERMINER LA POSITION D'UN ELEMENT DETECTEUR

(84) Benannte Vertragsstaaten:
**AT BE CH CY DE DK ES FI FR GB GR IE IT LI LU MC NL PT SE TR**

(30) Priorität: **26.07.2000 CH 147500**

(43) Veröffentlichungstag der Anmeldung:
**23.04.2003 Patentblatt 2003/17**

(73) Patentinhaber: **Northern Digital Inc.
Waterloo, Ontario N2V 1C5 (CA)**

(72) Erfinder:
• **SEILER, Paul, G.
CH-5234 Villigen (CH)**

• **MUENCH, Ralph, K.
CH-8108 Oberflachs (CH)**
• **KIRSCH, Stefan
79787 Lauchringen (DE)**

(74) Vertreter: **Rigling, Peter Daniel
Troesch Scheidegger Werner AG
Schwäntenmos 14
8126 Zumikon (CH)**

(56) Entgegenhaltungen:
**EP-A- 0 993 804          WO-A-01/33231**

**Beschreibung**

Die vorliegende Erfindung betrifft ein Verfahren nach dem Oberbegriff des Patentanspruchs 1, eine Anwendung des Verfahrens, eine Vorrichtung zur Durchführung des Verfahrens sowie ein Computerprogrammprodukt.

Bei zahlreichen technischen und medizinischen Verfahren ist die genaue Kenntnis der Position eines bestimmten Gegenstandes von entscheidender Bedeutung. Währenddem in der Medizin die Position von einzelnen Gewebeteilen - beispielsweise eines Tumors, der zur Zerstörung oder zur Wachstumsbegrenzung bestrahlt werden soll - bestimmt werden muss, ist die Positionserfassung zur Eingabe in ein Computersystem, beispielsweise für "Cyber Space"-Anwendungen, von allgemeiner Bedeutung. Eine solche Positionserfassungs- bzw. Positionseingabeeinheit wird in diesen Anwendungen auch etwa als dreidimensionale Maus bezeichnet.

Eine bekannte Vorrichtung bzw. ein bekanntes Verfahren zur Bestimmung der Position ist in der internationalen Patentanmeldung mit der Veröffentlichungsnummer WO 97/36192 der gleichen Anmelderin beschrieben. Gemäss der bekannten Lehre ist es vorgesehen, ein Wechselfeld mit Hilfe einer Feldgeneratoreinheit aufzubauen, wobei je nach der Anzahl Freiheitsgrade eines Sensorelementes, dessen Position bestimmt werden soll, mehrere Wechselfelder einander überlagert werden. Mit Hilfe einer Verarbeitungs- und Steuereinheit, welche die Feldgeneratoreinheit einerseits steuert, anderseits die vom Sensorelement empfangenen Signale verarbeitet, wird die Position und gegebenenfalls die Lage der Sensoreinheit bestimmt. Der Inhalt der vorstehend genannten Veröffentlichung bildet diesbezüglich integrierender Bestandteil dieser Beschreibung.

Es hat sich gezeigt, dass bei einer magnetfeldbasierten Ortung, wie sie beispielsweise bei der bekannten Lehre gemäss WO 97/36192 angewandt wird, in benachbarten, elektrisch leitenden Objekten Wirbelströme erzeugt werden. Diese führen zu Verzerrungen des ursprünglichen magnetischen Wechselfeldes und damit zu systematischen Fehlern. Das bedeutet, dass, wenn Position und Ausrichtung von Sensorelementen im verzerrten Wechselfeld so bestimmt werden, als wäre kein elektrisch leitendes Objekt zugegen, die gewonnenen Werte systematisch verfälscht sind.

Ein Verfahren zur Kompensation von Störeffekten, welche durch leitende Objekte hervorgerufen werden, ist unter dem Namen "Distortion Mapping" bekannt. Dieses Verfahren wird beispielsweise im Aufsatz mit dem Titel "Calibration of Tracking Systems in a Surgical Environment" (Birkfellner et. al., IEEE Trans Med Imaging, Vol. 17(5), Seiten 737 bis 742, 1998) beschrieben. Beim bekannten Verfahren wird die Position und die Orientierung eines Sensorelementes ebenfalls mit Hilfe eines Positionsmesssystems, das auf einer magnetfeldbasierten Ortung beruht, vorgenommen, wobei zur Kompensation von Störeffekten ein zweites Positionsmesssystems vorgesehen ist, welches durch elektrisch leitende Objekte nicht beeinflussbar ist. Die Differenz zwischen den mit den beiden Positionsmesssystemen ermittelten Positionen und Orientierungen werden in der Folge zur Korrektur der mit Hilfe des magnetfeldbasierten Positionsmesssystems bestimmten Position und Orientierung verwendet. Das bekannte Verfahren weist jedoch den Nachteil auf, dass, zur Erreichung einer hohen Genauigkeit, die Positions- und Orientierungsdifferenz an möglichst vielen Punkten gemessen werden muss. Um weitere Punkte zu erhalten, muss zusätzlich ein aufwendiges Interpolationsverfahren angewendet werden. Der sehr hohe Aufwand wird insbesondere durch das folgende Beispiel deutlich: Soll ein Volumen von 1 $m^3$ vermessen werden, wobei dies in den drei Achsen alle 10cm und in zehn verschiedenen Orientierungswinkeln geschehen soll, so erhält man 10'000 Punkte. Darüber hinaus ist das erwähnte zweite Positionsmesssystem erforderlich.

Des weiteren ist ein verfahren zur Kompensation von Störeffekten bekannt, bei dem Magnetfelder durch gepulste Gleichstromfelder erzeugt werden, wobei die Kompensation von Wirbelstromeffekten dadurch vorgenommen werden, indem Magnetfeldmessungen erst nach dem Abklingen der im Messsignal enthaltenen Wirbelstromanteile durchgeführt werden. Weiterführende Erläuterungen zum bekannten Verfahren können den Druckschriften US-5 453 686 und US-5 767 669 entnommen werden. Es hat sich gezeigt, dass die Genauigkeit der ermittelten Resultate ungenügend ist. Insbesondere ist die Kompensation unvollständig, wenn die Zerfallszeiten der Wirbelstromanteile die Pulszeit zwischen zwei Gleichstrompulsen übersteigt. Zwar kann dem durch Verlängerung der Pulszeit begegnet werden, doch führt dies zu einer unerwünschten geringeren Messrate. Des weiteren lässt sich die bekannte Kompensationsmethode nicht bei auf magnetischer Ortung basierenden Positionsmesssystemen anwenden, die magnetische Wechselfelder erzeugen.

Die Europäische Patentanmeldung mit der Veröffentlichungsnummer EP 0 993 804 offenbart ein Verfahren und eine Vorrichtung zur Verfolgung eines Objekts mittels eines Energiefeldes in der Gegenwart von Interferenz verursacht durch die Einführung eines Gegenstandes, welcher auf das Energiefeld anspricht, in die Nähe des Objekts.

Der vorliegenden Erfindung liegt daher die Aufgabe zugrunde, ein Verfahren anzugeben, das eine verbesserte Bestimmung der Position und/oder der Lage eines Sensorelementes ermöglicht.

Diese Aufgabe wird durch die im kennzeichnenden Teil des Patentanspruchs 1 angegebenen Massnahmen gelöst. Vorteilhafte Ausgestaltungen der Erfindung, eine Anwendung des Verfahrens, eine Vorrichtung zur Durchführung des Verfahrens sowie ein Computerprogrammprodukt sind in weiteren Ansprüchen angegeben.

Mit dem erfindungsgemässen Verfahren wird es ermöglicht, den Einfluss von leitenden Objekten zu eliminieren, zumindest aber erheblich zu reduzieren. Des weiteren ist dieses Verfahren allgemeiner und genauer als die bekannten Verfahren. Schliesslich kann der geometrieabhängige Teil der Berechnungen im Sinne einer Systemkalibrierung vor dem eigentliche Einsatz des Positionsmesssystems vorgenommen werden.

Die Erfindung wird nachfolgend anhand von Zeichnungen beispielsweise näher erläutert. Dabei zeigen

Fig. 1 eine bekannte Anordnung, bestehend aus Feldgeneratoreinheit, Sensorelement und Verarbeitungs- und Steuereinheit, in schematischer Darstellung, mit einem elektrisch leitenden Objekt,

Fig. 2 ein elektrisch leitendes Objekt und

Fig. 3 ein Flussdiagramm mit einigen Verfahrensschritten des erfindungsgemässen Verfahrens.

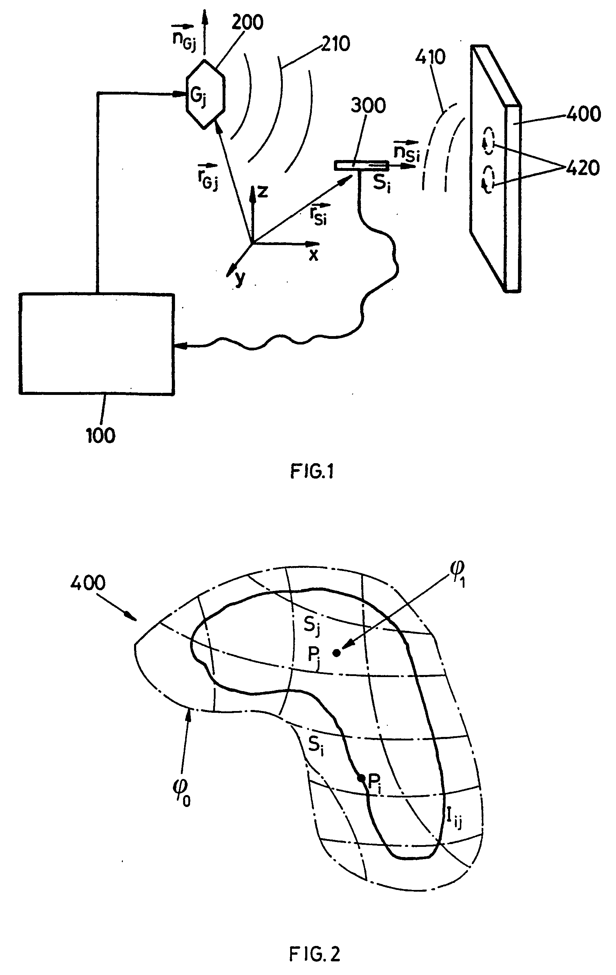

In Fig. 1 ist eine bekannte Anordnung, bestehend aus einer Feldgeneratoreinheit 200, einem Sensorelement 300 und einer Verarbeitungs- und Steuereinheit 100, dargestellt. Die Verarbeitungs- und Steuereinheit 100 ist jeweils über Leitungen mit der Feldgeneratoreinheit 200 einerseits und dem Sensorelement 300 anderseits verbunden. Während sich die Feldgeneratoreinheit 200 vorzugsweise an einem bekannten Ort befindet - was bedeutet, dass die Koordinaten x, y, z, inkl. der Ausrichtung im Koordinatensystem, bekannt sind - kann das Sensorelement 300 beliebig bewegt werden bzw. eine beliebige Position und Orientierung einnehmen. Es wird darauf hingewiesen, dass es denkbar ist, wie bereits aus der WO 97/36192 bekannt ist, dass das Sensorelement 300 ortsfest und die Feldgeneratoreinheit 200 frei, d.h. im Rahmen der zur Verfügung gestellten Verbindungsleitung zur Verarbeitungs- und Steuereinheit 100, beweglich ist. Des weiteren ist auch ohne weiteres denkbar, dass die Verarbeitungs- und Steuereinheit 100 in mehreren Funktionseinheiten realisiert ist, wie zum Beispiel, dass die Steuereinheit zur Steuerung der Feldgeneratoreinheit 200 in einem Funktionsblock und die Verarbeitungseinheit, in der die eigentliche Positionsberechnung der Position des Sensorelementes 300 vorgenommen wird, in einem anderen Funktionsblock realisiert ist. Diese Abwandlungen von der in Fig. 1 dargestellten Anordnung haben keinen Einfluss auf die Anwendbarkeit des erfindungsgemässen Verfahrens. Das Gleiche gilt auch für Ausführungsformen, bei denen mehrere Feldgeneratoren an unterschiedlichen Stellen vorgesehen sind, wie dies beispielsweise bei der Lehre gemäss WO 97/36192 der Fall ist.

Mit 400 ist in schematischer Darstellung ein elektrisch leitendes Objekt stellvertretend für diejenigen Gegenstände dargestellt, welche die magnetische Ortung des Sensorelementes 300 stört, indem im Objekt 400 Wirbelströme 420 erzeugt werden, aufgrund derer ein dem Wechselfeld 210 überlagerten Störfeld 410 entsteht.

Bevor auf das erfindungsgemässe Verfahren weiter beschrieben wird, werden im folgenden zunächst allgemeine Zusammenhänge bzw. Vorgehensweisen bei der magnetfeldbasierten Ortung erläutert.

Wie bereits erwähnt wurde, wird bei der magnetfeldbasierten Ortung, welche auch etwa als magnetische Ortung bezeichnet wird, die Position und/oder Ausrichtung von einem oder mehreren Sensorelementen 300 relativ zu einem oder mehreren Feldgeneratoreinheiten 200 bestimmt. Die Position $\vec{r}_{s_i}$ und Orientierung $\vec{n}_{s_i}$ der Sensorelemente $S_i$ kann durch Lösung des folgenden Gleichungssystems bestimmt werden, wenn man voraussetzt, dass Position $\vec{r}_{G_j}$ und Orientierung $\vec{n}_{G_j}$ der Feldgeneratoreinheiten $G_j$ bekannt sind:

$$F_{ij} = F(\vec{r}_{s_i}, \vec{n}_{s_i}, \vec{r}_{G_j}, \vec{n}_{G_j}) \tag{1}$$

Mit i ist das i-te Sensorelement und mit j die j-te Feldgeneratoreinheit gemeint. F ist dabei eine vom Magnetfeld abhängige Messfunktion von meistens einer Komponente des Magnetfelds $\vec{B}(x,y,z,t)$ (z.B. die induzierte spannung in einer Sensorspule). F kann natürlich auch eine Funktion von vielen zusammengebauten Sensoren in einem Sensorelement sein, welches mehrere oder alle Komponenten gleichzeitig misst.

Nach Art der Lösung dieses Gleichungssystems können magnetische Positionssysteme in zwei Klassen unterteilt werden:

I. Das Gleichungssystem wird invertiert, d.h. die Sensorelementpositionen können aus den gemessenen Magnetfeldern berechnet werden:

$$\vec{r}_{s_i} = f_r(F_{ij}) \ und \ \vec{n}_{si} = f_n \ (F_{ij}) \tag{2}$$

Da die Inversion des Gleichungssystems nur in sehr speziellen Fällen möglich ist, kann man durch Näherung versuchen, die Feldgleichungen in eine invertierbare Form zu bringen.

II. Das Gleichungssystem wird durch Optimierung gelöst, d.h. die Sensorelementpositionen werden so lange variiert, bis die nach Gleichung 1 berechneten Werte $F_{ij}$ am besten mit den gemessenen Werten $F_{ij}{}^M$ übereinstimmen. Eine mögliche Methode wäre ein Chi$^2$-Fit nach Levenberg-Marquardt. Dabei werden die Sensorpositionen $\vec{r}_{S_i}$ und $\vec{n}_{S_i}$ so lange variiert bis

$$Chi^2(\vec{r}_{S_i},\vec{n}_{S_i}) = \sum_j \frac{(F_{ij} - F_{ij}{}^M)^2}{(\Delta F_{ij}{}^M)^2} \qquad (3)$$

minimal wird. Für weiterführende Angaben zur Methode nach Levenberg-Marquardt wird stellvertretend auf die Druckschrift mit dem Titel "Numerical Recipies in C" (W. H. Press, S. A. Teukolsky, W. T. Vetterling und B. P. Flannery; Cambridge university Press; 1994) verwiesen.

Es ist auch eine Kombination beider Lösungsansätze möglich.

Da die vom Sensorelement $S_i$ gemessene Grösse $F_{ij}$ nur von der relativen Lage vom Sensorelement $S_i$ und der Feldgeneratoreinheit $G_j$ abhängt, sind die Rollen von Sensorelement und Feldgeneratoreinheit in allen magnetischen Positionsmesssystemen austauschbar.

Werden zeitlich variierende magnetische Felder benützt, so erzeugen diese - wie bereits erwähnt wurde - in benachbarten, elektrisch leitenden Objekten 400 Wirbelströme 420. Diese führen zu Verzerrungen des ursprünglichen magnetischen Wechselfeldes 210 und damit zu systematischen Fehlern bei der Positionsbestimmung. Das bedeutet, dass, wenn Position und Ausrichtung von Sensorelementen im verzerrten Wechselfeld so bestimmt werden, als wäre kein elektrisch leitendes Objekt 400 zugegen, die gewonnenen Werte systematisch verfälscht sind.

Um das Verfahren der magnetischen Ortung auch in der Nachbarschaft von elektrisch leitenden Objekten 400 frei von durch diese hervorgerufenen Fehlern anwenden zu können, werden erfindungsgemäss die Wechselfeldverzerrungen und ihr Einfluss auf die Bestimmung von Sensorelementposition und Sensorelementausrichtung bestimmt. Damit können die auftretenden systematischen Fehler korrigiert werden, womit die Genauigkeit der Position und/oder der Ausrichtung erheblich verbessert werden kann.

Bei einer vorgegebenen Messanordung hat man feststellen können, dass ein Messfehler von 4cm mit dem erfindungsgemässen Verfahren auf weniger als 1,5mm reduziert werden konnte.

Grundsätzlich lassen sich diese Korrekturen auch mit Hilfe der Technik der finiten Elemente und den Gleichungen der Elektrodynamik finden. Die bevorzugte Ausführungsform des erfindungsgemässen Verfahrens zeichnet sich gegenüber der Methode der finiten Elemente weiter dadurch aus, dass eine massive Reduktion der Positionsberechung erreicht wurde, da vieles im Sinne einer Systemkalibration im voraus berechnet werden kann.

Im folgenden wird das erfindungsgemässe Verfahren erläutert, wobei der Einfachheit halber zunächst davon ausgegangen wird, dass das Objekt 400 aus einer elektrisch leitenden Platte, d.h. aus einer flachen und begrenzten Fläche, besteht. Objekte 400, die in der Richtung einer gedachten Linie von der Feldgeneratoreinheit 200 zum Objekt 400 eine relevante Ausdehnung (Tiefe) besitzen, können ebenfalls mit dem erfindungsgemässen Verfahren behandelt werden. Für diesen Zweck wird die der Feldgeneratoreinheit 200 zugewandte Seite durch eine Vielflächen-Struktur angenähert. Dies ist zulässig, da die Wirbelströme 420 nur geringfügig in die Oberfläche eindringen. Die Tiefe eines dreidimensionalen Objektes 400 ist deshalb nicht relevant. Bei der mathematischen Bestimmung des Störfeldes 410 wird daher das Objekt 400 durch eine Vielflächenstruktur im vorstehend genannten Sinne angenähert.

Im folgenden werden Überlegungen betreffend die Berechnung von Feldverzerrungen bei einer leitende Platte dargestellt. Die daraus resultierenden Ergebnisse können analog bei allgemeineren Objektformen angestellt werden.

Befindet sich in einem zeitlich veränderlichen Magnetfeld $\vec{B}_0(x,y,z,t)$ ein elektrisch leitendes Objekt 400, so werden Wirbelströme 410 (Fig. 1) in die Oberfläche des Objektes 400 induziert. Diese Wirbelströme 410 verursachen ein weiteres Magnetfeld $\vec{B}'(x,y,z,t)$, welches dem ursprünglichen Feld $\vec{B}_0(x,y,z,t)$ überlagert ist und zu einem resultierenden Feld $\vec{B}_{Res}(x,y,z,t)$ führt. $\vec{B}_{Res}(x,y,z,t)$ ist gegenüber dem Feld $\vec{B}_0(x,y,z,t)$ verzerrt. Um dieses verzerrte Feld berechnen zu können, ist es notwendig, das induzierte Wechselfeld $\vec{B}'(x,y,z,t)$ zu kennen. Es lässt sich aus dem Biot-Savart Gesetz der Elektrodynamik (Gleichung 4) ein Feld $\vec{B}_1(x,y,z,t)$ berechnen, welches gut genug $\vec{B}'(x,y,z,t)$ beschreibt, falls der örtliche und zeitliche Verlauf der Wirbelströme 410 im Objekt 400 in $N$ verschiedenen punktförmigen Stromelementen bekannt ist:

$$B_1(\vec{P}_R, t) = \sum_{i=0}^{N} \frac{\mu_0 \mu_r I_i(t)}{4\pi} \frac{\Delta\vec{s}(t) \times \vec{r}}{r^3} \qquad (4)$$

wobei $\vec{P}_R = (x,y,z)$ ein Punkt im Raum ist und der Vektor $\vec{r}$ vom Stromelement zum Punkt $\vec{P}_R$ zeigt. Vorfaktoren können, falls notwendig, eingeführt werden und/oder können im Betrag des Faktors $\Delta\vec{s}(t)$ enthalten sein. Eine detailliertere Berechnung von $\vec{B}_1(x,y,z,t)$ durch Einführung von Längsströmen oder Flächenströmen etc. anstatt Punktströmen ist möglich. Dadurch würde die Schreibweise von Gleichung 4 allerdings geändert werden. In den meisten Fällen kann die Gleichung 4 hingegen so übernommen werden, wie sie oben aufgeführt ist, falls $N$ gross genug gewählt wird.

Die Feldverzerrungen werden also in zwei Schritten berechnet. Der erste Schritt ist die Bestimmung der Wirbelströme 410 und der zweite Schritt ist die Berechnung eines durch die Wirbelströme 410 erzeugten Störfelds $\vec{B}_1(x,y,z,t)$, welches gut genug das Störfeld $\vec{B}'(x,y,z,t)$ beschreibt.

Fig. 2 zeigt das Objekt 400, das für die Bestimmung des Störfeldes 410 in eine Vielflächenstruktur, bestehend aus beliebig vielen Segmenten, aufgeteilt ist. Ausgehend von dieser Aufteilung und diversen weiteren Annahmen werden zunächst die Wirbelströme berechnet.

Die Wirbelströme fliessen an der Oberfläche des Objektes 400 mit einer für die Theorie "unwesentlichen" Eindringtiefe. Um das oben erwähnte Störfeld $\vec{B}'(x,y,z,t)$ hinreichend genau zu berechnen, reicht es aus, den zeitlichen Stromverlauf in einigen Punkten auf der Oberfläche des Objektes 400 zu kennen. Die Anzahl der Punkte hängt von der verlangten Genauigkeit ab. Die Wirbelströme werden also in Punkten berechnet, welche auf oder in der Nähe der Oberfläche des Objektes liegen.

In einem ersten Schritt wird das Objekt in $N$ beliebig geformte Segmente unterteilt, die sinnvollerweise (aber nicht notwendigerweise) das ganze Objekt abdecken. Die segmente werden nachfolgend zur eindeutigen Unterscheidung mit $S_i\{0 \leq i \leq N\text{-}1\}$ bezeichnet, wobei i als Index verwendet wird.

In einem zweiten Schritt wird pro Segment ein Stützpunkt $P_i$ gewählt. Es ist sinnvoll aber nicht zwingend, gleich viele Stützpunkte wie Segmente zu definieren und diese eindeutig zu den Segmenten zuzuordnen. Im folgenden wird der Einfachheit halber davon ausgegangen, dass $N$ Segmente $S_i$ mit je einem eindeutig zugeordneten Stützpunkt $P_i$ definiert werden. Die Stromdichte $\vec{i}_i(t)$ im Stützpunkt $P_i$ eines jeden Segments $S_i$ wird mittels der folgenden Formel berechnet:

$$\vec{i}_i(t) = \sum_{j=0}^{N} \vec{i}_{ij}(t) \; mit \; j <> i \qquad (5)$$

wobei $\vec{i}_{ij}(t)$ die Stromdichte des Wirbelstroms $I_{ij}(t)$ ist, welche durch die Flussänderung des Feldes von $\vec{B}_0(x,y,z,t)$ im Segment $S_i$ verursacht wird und durch den Stützpunkt $P_i$ oder in der Umgebung des Stützpunktes $P_i$ fliesst. Die Berechnung der einzelnen Wirbelströme $I_{ij}(t)$ ist im nächsten Abschnitt beschrieben. Zunächst gilt:

$$\vec{i}_{ij}(t) = \frac{\vec{\lambda} I_{ij}(t)}{A_s} \qquad (5a)$$

mit $\vec{\lambda}$ als Richtungsvektor (oder eine dazu fast kolineare Richtung) der Stromlinie durch den Stützpunkt $P_i$ im Stützpunkt $P_i$ und mit $A_s$ die Querschnittsfläche der Stromline ist, wobei:

$$A_s = \pi \cdot r^2(h) \qquad (5b)$$

mit

r = Radius der kreisrunden Querschnittsfläche;
h = Eindringtiefe;

Sind nun die Stromdichten $\vec{i}_i(t)$ bekannt, so ist auch $\vec{B}_1(x,y,z,t)$ als Störfeld verursacht durch $\vec{B}_0(x,y,z,t)$ bere-

chenbar - dazu kann $\vec{i}_i(t).A(S_i)$ direkt in die Gleichung 4 eingesetzt werden, wobei $A(S_i)$ die Fläche des Segments $S_i$ ist. $\vec{B}_1(x,y,z,t)$ kann in den meisten Fällen als $\vec{B}'(x,y,z,t)$ betrachtet werden. Als Effekt zweiter Ordnung kann an dieser Stelle $\vec{B}_1(x,y,z,t)$ als Urfeld eingesetzt werden, um wiederum Wirbelströme für ein zweites Störfeld $\vec{B}_2(x,y,z,t)$ zu berechnen (Einfluss der Wirbelströme auf einander), welches mit $\vec{B}_0(x,y,z,t)$ und $\vec{B}_1(x,y,z,t)$ überlagert ist. In zweiter Näherung wäre also $\vec{B}'(x,y,z,t)$ gleich der Summe aus $\vec{B}_1(x,y,z,t)$ und $\vec{B}_2(x,y,z,t)$ - diese iterative Vorgehensweise kann für Effekte beliebiger Ordnung fortgesetzt werden. Es ist jedoch in den meisten Anwendungen der Effekt erster Ordnung hinreichend genau.

Ein einzelner Wirbelstrom $I_{ij}(t)$ ist eine Stromlinie, die durch den Stützpunkt $P_i$ fliesst und durch die zeitliche Flussänderung des Feldes durch den Stützpunkt $P_j$ verursacht wird. Um $I_{ij}(t)$ zu berechnen sind die Induktivität $L_{ij}$, sein Ohm'scher Widerstand $R_{ij}$ und die zeitliche Flussänderung $\frac{d\Phi_j}{dt}$ notwendig. Sind diese Grössen bekannt, so ist $I_{ij}(t)$ durch die Lösung der Differentialgleichung

$$\frac{d\Phi_j}{dt} - L_{ij}\frac{dI_{ij}(t)}{dt} - R_{ij}I_{ij}(t) = 0 \tag{6}$$

gegeben. In vielen Fällen mag $\vec{B}_0(x,y,z,t)$ zeitlich periodisch sein oder sogar harmonisch oszillieren, dies ist aber für die Gültigkeit der erfindungsgemässen Methode keine Notwendigkeit.

Die Induktivität $L_{ij}$ und der Ohm'sche Widerstand $R_{ij}$ sind durch die geometrische Form des Wirbelstroms $I_{ij}(t)$ gegeben und die Flussänderung $\frac{d\Phi_j}{dt}$ durch das Feld $\vec{B}_0(x,y,z,t)$ an der Stelle $P_j$ zusammen mit der Fläche des Segments $S_j$. In den folgenden Schritten wird zuerst die Form des Wirbelstroms beschrieben, und anschliessend daraus die Induktivität $L_{ij}$ und der Ohm'sche Widerstand $R_{ij}$ berechnet.

Man stelle sich vor, dass eine einzelne Magnetfeldlinie $B$ durch eine kleine Fläche $dA$ um einen Punkt $P$ auf dem Objekt dringt. In diesem Fall wären die induzierten Stromlinien in der Nähe vom Punkt $P$ kreisförmig und am Rande des Objektes 400 würden sie der Begrenzung folgen, also die Form der Umrandung des Objekts haben. Die Form eines beliebigen Wirbelstroms ist ein Strom entlang einer Höhenlinie einer Fläche, welche die Potentialgleichung

$$\Delta\varphi = \frac{\partial^2\varphi}{\partial x^2} + \frac{\partial^2\varphi}{\partial y^2} = 0 \tag{7}$$

erfüllt, wobei $\varphi(x,y)$ das Potential darstellt. Die Randbedingungen zur eindeutigen Lösung von Gleichung 7 (ermitteln von $\varphi(x,y)$) sind aus Figur 2 zu entnehmen (nämlich $\phi_0$ am Rand des Objektes und $\phi_1$ im Punkt $P_j$, $\phi_0 <> \phi_1$). Diese Potentialgleichung ist am besten numerisch zu lösen. Mit der Form des Wirbelstroms und der Eindringtiefe h des Stroms in das Material ist die einzelne Stromlinie als Leiterschleife mit einem ringförmigen Materialquerschnitt mit dem Durchmesser der Eindringtiefe betrachtbar (andere sinnvolle Materialquerschnittsgeometrien sind natürlich denkbar, ändern aber an den Berechnungen nichts wesentliches).

Der Ohm'sche Widerstand der Leiterschleife wird somit zu

$$R = \frac{l\rho}{\left(\frac{h}{2}\right)^2 \pi} \tag{8}$$

mit

R = Ohmscher Widerstand [ Ω ]
l = Länge der Leiterschleife [ m ]
h = Eindringtiefe des Stroms [ m ]
ρ = spezifischer elektrischer Widerstand des Materials [ Ωm ]

Die Induktivität der Leiterschleife ist gegeben durch

$$L = \frac{2W}{i^2} \tag{9}$$

und kann numerisch berechnet werden, wobei

$$W = \int\limits_{Raum} \frac{\vec{B}^2}{2\mu_0} dV \qquad (10)$$

die gespeicherte Energie im vom Leiter erzeugten Magnetfeld bedeutet, falls ein Strom i im Leiter fliesst. Es gibt "beliebig" viele andere Näherungsformeln, welche Gleichungen (8), (9) und (10) ersetzen könnten und ähnliche Resultate liefern.

Für die Berechnung des Flusses $\Phi_j(t)$ aus dem Feld $\vec{B}_0(x,y,z,t)$ gilt:

$$\Phi_j(t) = \overrightarrow{B_0}(x,y,z,t) \cdot \vec{A}_j \qquad (11)$$

wobei $\vec{B}_0(x,y,z,t)$ das ungestörte Feld an der Stelle $P_j$ ist und $\vec{A}_j$ die Flächennormale des Segments $S_j$ ist, mit dem Betrag der Fläche des betreffenden Segments. Formel 11 ist eine Näherungsformel für die allgemein gültige Formel

$$\Phi_j(t) = \int\limits_{A_j} \vec{B}_0(x, y, z, t) d\vec{A} \qquad (11a)$$

und darf angewendet werden, wenn B über $A_j$ hinreichend homogen ist (so z.B. für kleine Flächen $A_j$). An dieser Stelle soll erwähnt werden, dass die Fläche $A_j$ des Segments $S_j$ nicht in allen Fällen vollständig innerhalb der Leiterschleife $l_{ij}(t)$ liegt - Korrekturen könnten diesbezüglich angebracht werden, sind aber in der Regel nicht notwendig.

Die teilweise intensiven Berechnung der Induktivitäten $L_{ij}$ und der Ohm'schen Widerstände $R_{ij}$ können mittels der Formeln (8), (9) und (10) im voraus berechnet werden, da diese allein von der Geometrie und des Materials des Objektes abhängig sind - dies im Sinne einer Systemkalibration. Einsetzen und Lösen der Formeln (4) und (5), um $\vec{B}_1$ $(x,y,z,t)$ zu berechnen, können zu einem anderen Zeitpunkt erfolgen, insbesondere wenn das Erregerfeld $\vec{B}_0(x,y,z,t)$ bekannt ist. Iterationen, wie z.B. Einsetzen des Feldes $\vec{B}_1(x,y,z,t)$ zur Berechnung eines Feldes $\vec{B}_2(x,y,z,t)$ usw., sind möglich. Solche Iterationen könnten aber auch im voraus erfolgen, indem man sie in die Induktivitäten $L_{ij}$ einbindet. Dies macht aber erst einen Sinn, wenn regelmässig Korrekturen höherer Ordnung benötigt werden.

In der Praxis werden bei der magnetischen Ortung Lage und Ausrichtung eines oder mehrerer Sensorelemente 300 (Fig. 1) in einem Magnetfeld bestimmt, das von einem oder mehreren Feldgeneratoreinheiten 200 erzeugt wird. In dem verwendeten Koordinatensystem ist die Position der Feldgeneratoreinheit 200 oder den Feldgeneratoreinheiten bekannt. Im Fall von magnetischen Wechselfeldern erzeugen benachbarte elektrisch leitende Objekte 400 Feldverzerrungen durch in den Objekten 400 induzierte Wirbelströme 420. Das erfindungsgemässe Verfahren zur Korrektur dieser Verzerrungen, dessen theoretische Grundlagen oben angegeben wurden, wird folgendermassen angewendet: Die Lage der elektrisch leitenden Objekte 400 im oben erwähnten Koordinatensystem ist bekannt oder wird durch Vermessung bestimmt. Die Objektkoordinaten werden in einem Computerprogramm, das zur Berechnung der Wirbelströme 420 und der daraus resultierenden Feldverzerrungen benutzt wird, derartig eingegeben, dass die in den oben angegebenen Formeln verwendeten Ortskoordinaten im durch die Feldgeneratoreinheit 200 definierten Koordinatensystem definiert sind. Mit dem Computerprogramm wird dann das durch die Wirbelströme 420 erzeugte Störfeld berechnet. Bei Berücksichtigung der Wirbelströme 420 ändert sich das Gleichungssystem 1 wie folgt:

$$F_{ij} = F(\vec{r}_{S_i}, \vec{n}_{S_i}, \vec{r}_{G_j}, \vec{n}_{G_j}) + \sum_{k=1}^{P} F_k'(\vec{r}_{S_i}, \vec{n}_{S_i}, \vec{r}_{G_j}, \vec{n}_{G_j}) \qquad (12)$$

wobei $F_{ij}'$ die durch den Wirbelströme 420 des Objektes k erzeugte Störung darstellt. P ist die Anzahl der Objekte. Wie diese Korrektur angewendet wird, hängt von der Art des magnetischen Positionsmesssystems ab.

I. Bei Systemen, die auf Gleichung 2 beruhen, werden die Messwerte iterativ korrigiert, d.h. man berechnet zuerst die ungestörte Lösung nach Gleichung 2. Mit der gefundenen Position des Sensorelementes 300 kann der Korrekturterm F' berechnet und von den Messungen $F_{ij}{}^M$ abgezogen werden. Mit den korrigierten Messungen wird wieder eine Position berechnet. Dieser Algorithmus wird fortgesetzt, bis die Variation der berechneten Positionen unter gewissen Toleranzschwellen liegen.

II. Bei Systemen, die auf Gleichung 3 beruhen, muss der Lösungsalgorithmus nicht geändert werden. In der Chi$^2$-Summe werden anstatt des Modells zur Berechnung der wirbelstromfreien Magnetfeldes $F_{ij}$ nach Gleichung 1 das Magnetfeld mit Wirbelstromkorrekturen nach Gleichung 12 benutzt.

III. Unter bestimmten Voraussetzungen kann es auch möglich sein, das Gleichungssystem 12 zu invertieren, was dann zu einer Lösung entsprechend Gleichung 2 führt.

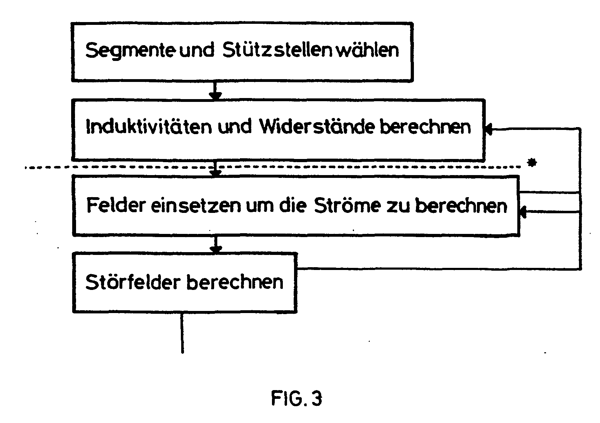

Fig. 3 zeigt, in vereinfachter Darstellung, ein Struktogramm eines nach dem erfindungsgemässen Verfahren arbeitenden Computerprogramms. Die einzelnen Verarbeitungsschritte wurden bereits anhand der Fig. 1 und 2 ausführlich erläutert.

Das erfindungsgemässe Verfahren kann auch vorzüglich für Objekte mit Öffnungen (Löcher) angewendet werden, wobei die Anzahl L der Öffnungen beliebig sein kann. Dabei muss für die schon beschriebene Lösungsmethode zunächst die Randbedingung der Potentialgleichung (7) an den Rändern der Öffnungen gleich dem Potential $\phi_0$ am Rande des Objektes sein. Des weiteren kommen zu der beschriebenen Methode weitere N mal L (N=Anzahl Stützpunkte und L=Anzahl Öffnungen) Stromlinien $I_{ik}$ hinzu, die in der Summe (5) noch zu addieren sind (k wandert von 1 bis L).

Die zusätzlichen Wirbelstromlinien $I_{ik}$ sind einzeln analog zu den Wirbelstromlinien $I_{ij}$ berechenbar, d.h. Lösen der Potentialgleichung (7) für die Form des Stroms und berechnung der Induktivität und des Widerstands nach Gleichungen (8) und (9). Bei der Potentialgleichung (7) ist allerdings zu beachten, dass die Randbedingung nicht "$\phi_1$ im Punkt Pj" lautet, sondern "$\phi_1$ am Rand der Öffnung k". Bei grossen Öffnungen ist eventuell Formel 11a für die Berechnung des Flusses zu verwenden anstatt die Näherungsformel 11.

Einzelne Leiterschleifen können auch mit dieser Methode berechnet werden, da die vorher erwähnten Öffnungen beliebig nahe an der Umrandung der zu berechnenden Objekte ausgedehnt werden kann. Das einfachste Beispiel ist ein Kreisring, welches als Scheibe mit einer fast gleich grossen Öffnung betrachtet werden kann: In diesem Beispiel sind die Stromlinien $I_{ij}$ vernachlässigbar (die Stützpunkte könnten weggelassen werden) und es gibt nur ein $I_{ik}$ dessen Form durch den Kreisring gegeben ist. Falls die Stützpunkte weggelassen werden, ist das Feld $B_1$ durch das Linienintegral über die Gleichung 4 zu bestimmen.

Ein weiterer Aspekt besteht darin, dass Störeinflüsse von unbekannten Objekten abgeschirmt werden, indem man zwischen Feldgeneratoreinheit und Objekt eine leitende Platte vorsieht, wobei deren Grösse und Form als auch deren Position bekannt sind. Damit müssen zwar die Feldverzerrungen dieser Platte berücksichtigt werden, doch alle anderen elektrisch leitenden Objekte, welche sich auf der in Bezug auf die Feldgeneratoreinheit anderen Seite der Platte befinden, können wegen der Abschirmung unberücksichtigt bleiben.

**Patentansprüche**

1. Verfahren zur Bestimmung der Position eines Sensorelementes (300), mit Hilfe dessen ein von mindestens einer Generatoreinheit (200) ausgesendetes magnetisches Wechselfeld (210) gemessen wird, wobei aufgrund eines im Sensorelement (300) empfangenen Signals die Position des Sensorelementes (300) bestimmt wird, ferner, vorzugsweise in erster Näherung, Störfelder (410) berechnet werden, welche aufgrund von in.elektrisch leitenden Objekten (400) erzeugten Wirbelströmen (420) entstehen, und die Position, welche ausgehend vom im Sensorelement (300) empfangenen Signal bestimmbar ist, aufgrund der berechneten Störfelder (410) korrigiert wird, **dadurch gekennzeichnet,**

   - **dass** die Wirbelströme (420) im Objekt (400), ausgehend vom Wechselfeld (210), berechnet werden und
   - **dass** die Störfelder (410), ausgehend von den berechneten Wirbelströmen (420) berechnet werden.

2. Verfahren nach Anspruch 1, **dadurch gekennzeichnet, dass** zur weiteren Verbesserung der Positionsbestimmung mindestens eine weitere Iteration durchgeführt wird, indem

   - weitere Wirbelströme im Objekt (400), ausgehend von berechneten Störfeldern (410), berechnet werden und
   - weitere Störfelder, ausgehend von den weiteren Wirbelströmen, berechnet werden.

3. Verfahren nach einem der vorangehenden Ansprüche,

**dadurch gekennzeichnet, dass** Position und Form der Objekte (400) bestimmt werden und dass Widerstände nach der Gleichung

$$R = \frac{l\rho}{\left(\frac{h}{2}\right)^2 \pi}$$

und Induktivitäten nach der Gleichung

$$L = \frac{2W}{i^2}$$

im Objekt (400) berechnet werden.

4. Verfahren nach Anspruch 3, **dadurch gekennzeichnet, dass** zur weiteren Verbesserung der Induktivitäten eine weitere Iteration durchgeführt wird, indem

   - weitere Wirbelströme im Objekt (400), ausgehend von berechneten Störfeldern (410), berechnet werden,
   - weiter Induktivitäten, ausgehend von den weiteren Wirbelströmen, bestimmt werden.

5. Verfahren nach Anspruch 3 und 4, **dadurch gekennzeichnet, dass** die Bestimmung der Objekte (400), die Bestimmung der Widerstände und Induktivitäten im Objekt (400) im Sinne einer Systemkalibrierung im voraus, d.h. vor den das Wechselfeld (210) berücksichtigenden Berechnungen, bestimmt werden.

6. Verfahren nach einem der vorangehenden Ansprüche,
   **dadurch gekennzeichnet, dass** zur Bestimmung der Wirbelströme wie folgt vorgegangen wird:

   - die Objekte (400) werden in Segmente ($S_i$) und Stützstellen (Pi) aufgeteilt;
   - in den Stützpunkten (Pi) wird die Stromdichte ($i_i$) bestimmt;
   - aus den Wirbelströmen ($I_{ij}$) werden die Stromdichten ($i_i$) bestimmt.

7. Anwendung des Verfahrens nach einem der Ansprüche 1 bis 6 für die magnetfeldorientierte Ortung bei "Cyber Space"-Anwendungen.

8. Vorrichtung zur Durchführung des Verfahrens nach Anspruch 1 bis 6, wobei mindestens eine Feldgeneratoreinheit (200) zur Aussendung eines magnetischen Wechselfeldes (210), mindestens ein Sensorelement (300), mit Hilfe dessen ein von der mindestens einen Feldgeneratoreinheit (200) ausgesendetes magnetisches Wechselfeld (210) messbar ist, wobei aufgrund eines im Sensorelement (300) empfangenen Signals die Position des Sensorelementes (300) bestimmbar ist, und eine Verarbeitungs- und Steuereinheit (100), mit Hilfe deren, vorzugsweise in erster Näherung, Störfelder (410) berechenbar sind, welche aufgrund von in elektrisch leitenden Objekten (400) erzeugten Wirbelströmen (420) entstehen, und die Position, welche ausgehend vom im Sensorelement (300) empfangenen Signal bestimmbar ist, aufgrund der berechneten Störfelder (410) korrigierbar ist vorgesehen sind, **dadurch gekennzeichnet, dass** die Feldgeneratoreinheit (200) und das Sensorelement (300) mit der Verarbeitungs- und Steuereinheit (100) verbunden sind und mit Hilfe der Verarbeitungs- und Steuereinheit (100) die Wirbelströme (420) im Objekt (400), ausgehend vom Wechselfeld (210), berechenbar sind und die Störfelder (410), ausgehend von den berechneten Wirbelströmen (420) berechenbar sind.

9. Vorrichtung nach Anspruch 8, **dadurch gekennzeichnet, dass** mindestens ein elektrisch leitendes Objekt zum Abschirmen der Feldgeneratoreinheit (200) vorgesehen sind.

10. Computerprogrammprodukt, das in den internen Speicher eines digitalen Computers geladen werden kann und Softwarecodeabschnitte umfasst, mit denen die Schritte gemäss den Ansprüchen 1 bis 6 ausgeführt werden, wenn das Produkt auf einem Computer läuft.

**Claims**

1. Method for determining the position of a sensor element (300) with the aid of which a magnetic alternating field (210) emitted by at least one generator unit (200) is measured, the position of the sensor element (300) being determined on the basis of a signal received in the sensor element (300), further, preferably in first approximation, disturbing fields (410) being calculated which result from eddy currents (420) generated in electric conductive objects (400), and the position which is determinable from the signal received in the sensor element (300), being corrected on the basis of the calculated disturbing fields (410), **characterized in**

   - **that** the eddy currents (420) in the object (400) are calculated on a basis of the alternating field (210), and
   - **that** the disturbing fields (410) are calculated on a basis of the calculated eddy currents (420).

2. Method according to claim 1, **characterized in that** at least one further iteration is carried out for further improving the position determination by

   - calculating further eddy currents in the object (400) on a basis of the calculated disturbing fields (410), and
   - calculating further disturbing fields on a basis of the further eddy currents.

3. Method according to one of the preceding claims, **characterized in that** the position and the form of the objects (400) are determined and that resistances in the object (400) are calculated by the equation

$$R = \frac{l\rho}{\left(\frac{h}{2}\right)^2 \pi}$$

   and inductivities in the object (400) are calculated by the equation

$$L = \frac{2W}{i^2}$$

4. Method according to claim 3, **characterized in that** a further iteration is carried out for further improving the inductivities by

   - calculating further eddy currents in the object (400) on a basis of the calculated disturbing fields (410), and
   - determining further inductivities on a basis of the further eddy currents.

5. Method according to claim 3 and 4 **characterized in that** the determination of the objects (400), the determination of the resistances and inductivities in the object (400) are determined in the sense of a system calibration in advance, i.e. before the calculations, which take into account the alternating field (210).

6. Method according to one of the preceding claims, **characterized in that** one proceeds as follows for determining the eddy currents:

   - the objects (400) are divided into segments ($S_i$) and supporting points ($P_i$);
   - the current density ($i_i$) is determined in the supporting points ($P_i$);
   - the current densities ($i_i$) is determined from the eddy currents ($I_{ij}$).

7. Use of the method according to one of the claims 1 to 6 for the magnetic field oriented localization in "Cyber Space" applications.

8. Apparatus for carrying out the method according to claim 1 to 6, at least a field generator unit (200) for emitting a magnetic alternating field (210), at least a sensor element (300) with the aid of which a magnetic alternating field (210) emitted by the at least one field generator unit (200) is measured, the position of the sensor element (300) being determined on the basis of a signal received in the sensor element (300), and a processing and control unit (100) with the aid of which, preferably in first approximation, disturbing fields (410) are calculable that result from eddy currents (420) generated by electric conductive objects (400), and the position, which is determinable on the basis of the signal received in the sensor element (300), is correctable on the basis of the calculated disturbing fields (410), being provided, **characterized in that** the field generator unit (200) and the sensor element (300) are connected to the processing and control unit (100) and the eddy currents (420) in the object (400), resulting from the alternating field (210), are calculable with the aid of the processing and control unit (100) and the disturbing fields (410) are calculable from the calculated eddy currents (420).

9. Apparatus according to claim 8, **characterized in that** at least one electric conductive object for shielding the field generator unit (200) are provided.

10. Computer program product, which can be loaded into the internal memory of a digital computer and which comprises software code sections, by means of which the steps according to the claims 1 to 6 are carried out, when the product runs on a computer.

**Revendications**

1. Procédé pour déterminer la position d'un élément détecteur (300) à l'aide duquel est mesuré un champ magnétique alternatif (210) émis par au moins une unité générateur (200), dans lequel on détermine la position de l'élément détecteur (300) sur la base d'un signal reçu dans l'élément détecteur (300) et on calcule, de préférence par une première approximation des champs parasitaires (410) formés sur la base de courants parasites (420) produits dans des objets (400) électroconducteurs, et on corrige la position, qui peut être déterminée a partir du signal reçu dans l'élément détecteur (300), sur la base des champs parasites (410) calculés, **caractérisé en ce que**

   - les courants parasites (420) dans l'objet (400) sont calculés en partant du champ alternatif et

   - les champs parasitaires (410) sont calculés en partant des courants parasites (420) calculés.

2. Procédé selon la revendication 1, **caractérisé en ce que** pour perfectionner la détermination de la position on procède à une itération supplémentaire par le calcul d'autres courants parasites dans l'objet (400) en partant de champs parasitaires calculés (410) et par le calcul d'autres champs parasitaires partant desdits autres courants parasites.

3. Procédé selon l'une des revendications précédents, **caractérisé en ce que** la position et la forme des objets (400) sont déterminées et **en ce que** l'on calcule dans l'objet (400) des résistances selon l'équation

$$R = \frac{l\rho}{\left(\frac{h}{2}\right)^2 \pi}$$

et des inductances selon l'équation

$$L = \frac{2W}{i^2}$$

4. Procédé selon la revendication 3, **caractérisé en ce que** pour perfectionner les inductances on procède à une itération additionnelle par le calcul d'autres courants parasites dans l'objet (400) en partant de champs parasitaires calculés et par la détermination d'autres inductances en partant desdits autres courants parasites.

5. Procédé selon les revendications 3 et 4, **caractérisé en ce que** la détermination des objets (400) et la détermination des résistances et inductances dans l'objet (400) dans le sens d'un calibrage du système se fait à l'avance, c'est-à-dire avant les calculs tenant compte du champ alternative (210).

6. Procédé selon l'une des revendications précédents, **caractérisé en ce que** l'on procède comme suit pour déterminer les courants parasites:

   - les objets (400) sont séparés en segments ($S_i$) et points d'appui ($P_i$);

- on détermine dans les points d'appui ($P_i$) la densité de courant ($i_i$);

- on détermine à partir des courants parasites ($I_{ij}$) les densités de courant ($i_i$).

7. Application du procédé selon l'une des revendications 1 à 6 pour le repérage par champ magnétique orienté lors d'applications "Cyber Space".

8. Appareil pour la mise en oeuvre du procédé selon les revendications 1 à 6, comprenant au moins une unité générateur de champ (200) pour émettre un champ magnétique alternative (210), au moins un élément détecteur (300) à l'aide duquel peut être mesuré un champ magnétique alternative (210) émis par ladite unité générateur de champ (200), la position de l'élément détecteur (300) pouvant être déterminée à l'aide d'un signal reçu dans l'élément détecteur (300), et une unité de traitement et de commande (100) permettant, de préférence par une première approximation, de calculer des champs parasitaires (410) formés sur la base de courants parasites (420) produits dans des objets (400) électroconducteurs, et de corriger la position, qui peut être déterminée à partir du signal reçu dans l'élément détecteur (300), sur la base des champs parasites (410) calculés, **caractérisé en ce que** l'unité générateur de champ (200) et l'élément détecteur (300) sont reliés avec l'unité de traitement et de commande (100), les courants parasites (420) dans l'objet (400), pouvant être calculés à l'aide de l'unité de traitement et de commande (100) partant du champ alternative (210) et les champs parasites (410) pouvant être calculés partant des courants parasites (420) calculés.

9. Appareil selon la revendication 8, **caractérisé par** au moins un objet électroconductif pour protéger l'unité générateur de champ (200).

10. Produit de programme d'ordinateur qui peut être chargé dans la mémoire interne d'un ordinateur digital et que comprend des sections de code de software avec lesquelles les pas selon les revendications 1 à 6 sont mis en oeuvre lorsque le produit marche dans un ordinateur.

FIG.1

FIG. 2

13

Segmente und Stützstellen wählen

Induktivitäten und Widerstände berechnen

Felder einsetzen um die Ströme zu berechnen

Störfelder berechnen

FIG. 3